# EUROPEAN PATENT SPECIFICATION

(11) **EP 2 167 450 B1**
(45) Date of publication and mention of the grant of the patent: **16.03.2016**
(21) Application number: 08753967.2
(22) Date of filing: 26.05.2008
(51) Int. Cl.: C07C 29/86, C07C 31/22, C07C 31/24

(54) **METHOD FOR EXTRACTION IN A PROCESS FOR PRODUCTION OF A DI, TRI OR POLYHYDRIC ALCOHOL**
METHODE ZUR EXTRAKTION IN EINEM VERFAHREN ZUR HERSTELLUNG EINES ZWEI, DREI ODER MEHRERE HYDROXYLGRUPPEN ENTHALTENDEN ALKOHOLS
PROCÉDÉ D'EXTRACTION DANS UN PROCÉDÉ DE PRODUCTION D'UN ALCOOL DI, TRI OU POLYHYDRIQUE

(30) Priority: 18.06.2007 SE 0701478
(43) Date of publication of application: 31.03.2010
(73) Proprietor: Perstorp Specialty Chemicals AB, 284 80 Perstorp (SE)
(72) Inventor: PAJALIC, Oleg, S-291 66 Kristianstad (SE); ENGBERG, David, 281 53 Finja (SE); AXELSSON, Göran, S-243 35 Höör (SE)
(74) Representative: Hoffmann Eitle
(86) International application number: PCT/SE2008/000350
(87) International publication number: WO 2008/156406

(56) References cited:
- EP-A1- 1 848 679
- EP-A2- 1 620 362
- GB-A- 808 101
- GB-A- 816 208
- GB-A- 824 442
- JP-A- 54 144 307
- US-A- 2 436 209
- US-A- 2 629 746
- US-A- 3 076 854
- US-A- 4 482 768
- US-A- 6 080 896
- DATABASE WPI Week 197910, Derwent Publications Ltd., London, GB; AN 1979-19014B, XP008117886 & JP 54 012304 A (KURARAY CO LTD) 30 January 1979

## Description

The present invention refers to a method for extraction in a process for production of a di, tri or polyhydric alcohol selected from the group consisting of 2-alkyl-1,3-propanediols, 2,2-dialkyl-1,3-propanediols, 2-alkyl-2-hydroxyalkyl-1,3-propanediols and 2,2-dihydroxialkyl--1,3-propanediols, said method comprising feeding a mother liquid to a first extraction unit, extracting said mother liquid in at least one high boiling solvent, returning obtained raffinate to said process, feeding obtained extract to a second extraction unit, extracting said extract in water, obtaining a water phase comprising organic by-products and a solvent phase, and recycling said solvent.

US-B-3,076,854 concerns a process for recovering trimethylolpropane of high purity from aqueous reaction liquors which contain in addition to trimethylolpropane a large amount of various impurities.

EP-A-1 620 362 concerns a process for the recovery of 1,3-propanediol from an aqueous feed stream. The process involves contacting an aqueous feed stream that comprises water, 1,3-propanediol, and at least one contaminant with at least one solvent extractant to form a mixture. The mixture is separated into a first phase and a second phase. The second phase comprises a majority of the water from the aqueous feed stream. The first phase comprises solvent extractant and at least some of the 1,3-propanediol that was present in the aqueous feed stream. The weight ratio in the first phase of 1,3-propanediol to any one contaminant present is greater than the weight ratio of 1,3-propanediol to the same contaminant in the aqueous feed stream prior to the aqueous feed stream being contacted with the solvent extractant. The first phase can be removed from the separated second phase in order to recover the 1,3-propanediol.

GB-B-816,208 concerns a process for the separation of trimethylolpropane from an aqueous solution containing trimethylolpropane and sodium formate, which comprises subjecting said solution to treatment with a liquid solvent for trimethylolpropane so as to obtain an aqueous phase containing sodium formate in solution and a solvent phase comprising a solution of trimethylolpropane in the liquid solvent, said liquid solvent being one which dissolves little water and is of low solubility in said aqueous phase, separating the phases and recovering trimethylolpropane from the solvent phase.

Di, tri and polyhydric alcohols having for instance a neopentyl structure, such as neopentyl glycol, 2-ethyl-2-butyl-1,3 propanediol, trimethylolethane, trimethylolpropane, trimethylolbutane and pentaerythritol are normally synthesised in an alkali catalysed aldol condensation of formaldehyde and a second aldehyde yielding a methylolaldehyde. Said methylolaldehyde is in a subsequent step reduced to corresponding alcohol by means of a so called crossed Cannizzaro reaction with a further equivalent of formaldehyde in the presence of a strong base, such as an alkali or alkaline earth metal hydroxide and/or an amine. The crossed Cannizzaro reaction can alternatively be replaced by a catalytic hydrogenation. The synthesis as well as recovery of obtained reaction product normally yield by-products, such as formates and acetals, such as linear and cyclic formals.

By-products, such as formals, present in the mother liquid of said process are known to interfere with the crystallisation process of the main di, tri or polyhydric alcohol and a decreased amount in the mother liquid of such by-products implies increased capacity of the process. Certain formals are also known to cause fouling in evaporators, dryers and filter beds. For instance, when the cyclic formal of pentaerythritol is present the dense mass of the filter cake forces the filter bed to be run with a very thin layer of cake to make the filtration possible.

Different organometallic salts, such as formates of calcium, sodium, potassium and the like, are also present in the mother liquid. The formate present in the mother liquid depends on the catalyst used during the manufacture of the di, tri or polyhydric alcohol.

It has now quite unexpectedly been found that extraction in diisopropyl ether, 2-ethylhexanol, 2-propylheptanol, ethylacetate, octanol, butyl acetate, isopropyl acetate, methyl ethyl ketone and/or methyl isobutyl ketone, is a very effective way of reducing the amount of for instance linear and cyclic formals in mother liquids yielded in the production of 2-alkyl-1,3-propanediols, 2,2-dialkyl-1,3-propanediols, 2-alkyl-2-hydroxyalkyl-1,3-propanediols and/or 2,2-dihydroxialkyl-1,3-propanediols. Preferred solvents are for instance 2-propylheptanol and/or most suitably 2-ethylhexanol, which exhibits high distribution coefficients, low solubility in water, hydrolytic stability, a flash point of 77°C, which means that explosive zone classification can be avoided, low toxicity and low enviromnental impact.

The method, according to the present invention, for extraction in a process for production of a 2-alkyl-1,3-propanediol, 2,2-dialkyl-1,3-propanediol, 2-alkyl-2-hydroxyalkyl-1,3-propanediol or 2,2-dihydroxialkyl-1,3-propanediol, such as 2-methyl-1,3-propanediol, neopentyl glycol, 2-butyl-2-ethyl-1,3-propanediol, trimethylolethane, trimethylolpropane, trimethylolbutane or pentaerythritol, comprises the steps of
i) feeding a mother liquid, obtained in a process for production of a 2-alkyl-l,3-propanediol, a 2,2-dialkyl-1,3-propanediol, a 2-alkyl-2-hydroxyalkyl--1,3-propanediol or a 2,2-dihydroxialkyl-1,3-propanediol, at a controlled mass flow to a first extraction unit,
ii) extracting said mother liquid in said first extraction unit in at least one high boiling solvent effective in extracting organic by-products and being present in an amount of more than 0.2 times said mother liquid mass flow,
iii) obtaining a raffinate and an extract comprising said solvent and organic by-products,
iv) recycling said raffinate to said process,
v) feeding at a controlled mass flow obtained extract to a second extraction unit,
vi) extracting said extract in water being present in an amount of 1/10 to 1 times said extract mass flow,
vii) obtaining a water phase comprising said organic by-products and a solvent phase, and
viii) recycling said solvent to step (ii), and optionally
ix) evaporating said water yielding dry organic by-products.

Said controlled mass flow is in especially preferred embodiments of the present invention obtained by allowing said mother liquid and/or said extract to be fed via a buffer tank or similar and said raffinate is preferably recycled to said process via a buffer tank or similar. The use of buffer tanks allows efficient control of the mass flow through flow measurements.

Important factors in the extraction are the quantity of solvent used and the efficiency of the extraction unit. The quantity of solvent should as disclosed above be more than 0.2 times the mass flow of the mother liquid feed but most optimal is a solvent quantity of 1 to 4 times the mass flow of said feed. The efficiency of the extraction unit depends on how many theoretical stages it comprises. The temperature of the mother liquid feed is most suitably in the range of 20-100°C depending on the solvent used.

Column extractors are very suitable, however most types of extractors are possible to use, such as mixer-settler, Karr type, Scheibel type, Kühni type, packed column with different types of structured or random packings and the like. Salts extracted by the solvent can be reduced or eliminated by a washing section included as part of the first extraction unit or as a separate unit. After the first extraction the raffinate is recycled to the process, optionally through a buffer tank or similar.

Solvent recovery is performed as disclosed in steps (v) through (viii) above. The extract comprising solvent and organic by-products is re-extracted in the second extraction unit with a small quantity of water, about 1/10 to 1 times, the mass flow of the extract. The water extracts organic components and the solvent is recycled to the first extraction unit. The most unique feature here is the use of a high boiling solvent in combination with re-extraction instead of a low boiling solvent and evaporation. Using re-extraction instead of evaporation results in substantial energy savings. Water can optionally be evaporated thus yielding dry organic by-products.

Said organic by-products include in embodiments of the present invention at least one linear or cyclic formal of a said 2-alkyl-1,3-propanediol, 2,2-dialkyl-1,3-propanediol, 2-alkyl--2-hydroxyalkyl-1,3-propanediol or 2,2-dihydroxialkyl-1,3-propanediol and/or at least one organometallic salt, such as potassium, sodium and/or calcium formate.

Without further elaboration, it is believed that one skilled in the art can, using the preceding description, utilise the present invention to its fullest extent. The following preferred specific embodiments are, therefore, to be construed as merely illustrative and not limitative of the remainder of the disclosure in any way whatsoever. In the following Examples 1-3 illustrate embodiments and steps of the method according to the present invention.

### Example 1

A mother liquid yielded in a process for production of pentaerythritol was extracted with 2-ethylhexanol using a bench-top mixer-settler (Mixer-Settler Unit MSU 0.5, Metallextraktion AB, Sweden). The mixer-settler consisted of blocks that can be put together allowing construction of a series of mixer-settlers with variable number of stages. Each stage included a built-in heavier and lighter solvent flow transfer. The equipment used in this experiment consisted of four stages. The active mixer volume was 0.12 litre and the settler volume was 0.48 litre resulting in a total volume of 0.6 litre in each stage.

The mixer-settler was run in a counter current way. The two phases were pumped in on opposite sides of the mixer-settler using hose pumps. The heavier phase entered the mixing chamber through a pipe joint inlet and was mixed with the lighter phase from the next step that entered by gravity through a pipe joint inlet in the upper part of the chamber. The two phases were mixed and the partition of the solute between the solvents in the dispersion formed takes place. The dispersion from the mixing chamber entered the settling compartment through a centrally located port. After settling by gravity the lighter solvent left the settling compartment through a channel for direct gravity flow to an adjacent mixing chamber, or flowed out of the mixer-settler. The heavier solvent flowed under a fixed baffle into the outflow cavity where the heavier solvent was taken out through a hole in the jack leg screw. The total volume of heavier solvent flowed through the outlet channel into an adjacent mixing chamber or out of the mixer-settler. Since the flow rate of the lighter solvent was dominating, the heavier phase was dispersed in the continuous lighter phase. The solvent pump was calibrated to a flow of 89 g/min and the solution pump was calibrated to a flow of 60 g/min which give to a residence time of 15 min. and a phase ratio solvent to solution of 2:1. After approximately 75 min. samples was taken from both phases in all four settlers and analysed evidencing that cyclic formal of pentaerythritol was separated from pentaerythritol by extraction with 2-ethylhexanol. The extract comprised 0.03% by weight of pentaerythritol and 0.23% by weight of the cyclic formal of pentaerythritol.

### Example 2

A mother liquid obtained in a process for production of trimethylolethane was extracted in 2-ethylhexanol using an agitated extraction column (Kühni ECR60/70G) with an inner diameter of 60 mm. The number of agitated compartments was 70 with a height of 2450 mm and the free open area of the partition plates was 30%. The feed point of 2-ethylhexanol was at the bottom of the column and the feed point of said mother liquid was at the upper part of the column - 4 trials were performed. The extraction temperature was 75-90°C. The double jacketed column was heated by steam to maintain the temperature. The mother liquid comprised by weight 35.9% of trimethylolethane (TME), 35.9% potassium formate (KFa) and 23.9% of water.

| | Temperature | TME in extract % w/w | KFa in extract % w/w |
|---|---|---|---|
| Trial 1 | 87 | 0.07 | 0.17 |
| Trial 2 | 74 | 0.50 | 0.05 |
| Trial 3 | 75 | 0.32 | 0.02 |
| Trial 4 | 74 | 0.05 | 0.19 |

### Example 3

The extract, solvent comprising organic by-products, obtained in Example 1 was re-extracted in water using the same four stage bench top mixer-settler as in Example 1. The flow of the water was set to 20 g/min and the flow of the extract to 80 g/min. A total of 4350 g of the 2-ethylhexanol solution and 1130 g of water was used in the re-extraction. The extract and the water was cycled through the mixer-settler twice yielding a total run time of about three hours. Samples of both phases on all stages was taken after about two and a half hour and analysed evidencing that obtained water phase comprised said organic by-products.

## Claims

1. A method for extraction in a process for production of a 2-alkyl-1,3-propanediol, a 2,2-dialkyl-1,3-propanediol, a 2-alkyl-2-hydroxyalkyl-1,3-propanediol or a 2,2-dihydroxyalkyl-1,3-propanediol **characterised in, that** said method comprises the steps of:
i) feeding a mother liquid, obtained in a process for production of a 2-alkyl-1,3-propanediol, 2,2-dialkyl-1,3-propanediol, 2-alkyl-2-hydroxyalkyl-1,3-propanediol and/or 2,2-dihydroxyalkyl-1,3-propanediol, at a controlled mass flow to a first extraction unit;
ii) extracting said mother liquid in said first extraction unit in at least one high-boiling solvent, wherein the at least one high-boiling solvent is diisopropyl ether, 2-ethylhexanol, 2-propylheptanol, ethylacetate, octanol, butyl acetate, isopropyl acetate, methyl ethyl ketone and/or methyl isobutyl ketone and is present in an amount of more than 0.2 times said mother liquid mass flow;
iii) obtaining a raffinate and an extract comprising said solvent and organic by-products;
iv) recycling said raffinate to said process;
v) feeding at a controlled mass flow obtained extract to a second extraction unit;
vi) extracting said extract in water being present in an amount of 1/10 to 1 times said extract mass flow;
vii) obtaining a water phase comprising said organic by-products and a solvent phase; and
viii) recycling said solvent phase to step (ii), and optionally
ix) evaporating said water yielding dry organic by-products.

2. A method according to Claim 1 **characterised in, that** said mother liquid and/or said extract is fed via a buffer tank.

3. A method according to Claim 1 or Claim 2 **characterised in, that** said raffinate is recycled to said process via a buffer tank.

4. A method according to any of the Claims 1-3 **characterised in, that** said solvent is present in an amount of 1-4 times said mother liquid mass flow.

5. A method according to any of the Claims 1-4 **characterised in, that** said mother liquid is fed at a temperature of 20-100°C.

6. A method according to any of the Claims 1-5 **characterised in, that** said 2-alkyl-1,3-propanediol, 2,2-dialkyl-1,3-propanediol, 2-alkyl-2-hydroxyalkyl-1,3-propanediol or 2,2-dihydroxyalkyl-1,3-propanediol is 2-methyl-1,3-propanediol, neopentyl glycol, 2-butyl-2-ethyl-1,3-propanediol, trimethylolethane, trimethylolpropane, trimethylolbutane or pentaerythritol.

7. A method according to any of the Claims 1-6 **characterised in, that** said organic by-products include at least one linear or cyclic formal of a 2-alkyl-1,3-propanediol, a 2,2-dialkyl-1,3-propanediol, a 2-alkyl-2-hydroxyalkyl-1,3-propanediol or a 2,2-dihydroxyalkyl-1,3-propanediol.

8. A method according to any of the Claims 1-7 **characterised in, that** said organic by-products include organometallic salts, such as potassium, sodium and/or calcium formate.

9. A method according to any of the Claims 1-8 **characterised in, that** a washing section is included as part of the first extraction unit or as a separate unit.

## Patentansprüche

1. Extraktionsverfahren in einem Verfahren zur Herstellung eines 2-Alkyl-1,3-propandiols, eines 2,2-Dialkyl-1,3-propandiols, eines 2-Alkyl-2-hydroxyalkyl-1,3-propandiols oder eines 2,2-Dihydroxyalkyl-1,3-propandiols, **dadurch gekennzeichnet, dass** das Verfahren die Schritte umfasst:
(i) Zuführen einer Mutterlauge, die in einem Verfahren zur Herstellung eines 2-Alkyl-1,3-propandiols, eines 2,2-Dialkyl-1,3-propandiols, eines 2-Alkyl-2-hydroxyalkyl-1,3-propandiols oder eines 2,2-Dihydroxyalkyl-1,3-propandiols erhalten wurde, in einem gesteuerten Massendurchfluss zu einer ersten Extraktionseinheit;
(ii) Extrahieren der Mutterlauge in dieser ersten Extraktionseinheit in zumindest einem hochsiedenden Lösungsmittel, wobei das zumindest eine hochsiedende Lösungsmittel Diisopropylether, 2-Ethylhexanol, 2-Propylheptanol, Ethylacetat, Octanol, Butylacetat, Isopropylacetat, Methylethylketon und/oder Methylisobutylketon ist und in einer Menge von mehr als dem 0,2-fachen des Mutterlauge-Massendurchflusses vorhanden ist;
(iii) Erhalten eines Raffinats und eines Extrakts, umfassend das Lösungsmittel und organische Nebenprodukte;
(iv) Zurückführen des Raffinats zu diesem Verfahren;
(v) Zuführen des erhaltenen Extrakts bei einem gesteuerten Massendurchfluss zu einer zweiten Extraktionseinheit;
(vi) Extrahieren des Extrakts in Wasser, das in einer Menge von 1/10 bis dem 1-fachen des Extrakt-Massendurchflusses vorhanden ist;
(vii) Erhalten einer Wasserphase, die die organischen Nebenprodukte und eine Lösungsmittelphase umfasst; und
(viii) Zurückführen der Lösungsmittelphase zu Schritt (ii) und gegebenenfalls
(ix) Verdampfen des Wassers, wodurch trockene organische Nebenprodukte gewonnen werden.

2. Verfahren gemäss Anspruch 1, **dadurch gekennzeichnet, dass** die Mutterlauge und/oder der Extrakt über einen Pufferbehälter zugeführt wird.

3. Verfahren gemäss Anspruch 1 oder Anspruch 2, **dadurch gekennzeichnet, dass** das Raffinat über einen Pufferbehälter zu dem Verfahren zurückgeführt wird.

4. Verfahren gemäss einem der Ansprüche 1 bis 3, **dadurch gekennzeichnet, dass** das Lösungsmittel in der 1- bis 4-fachen Menge des Mutterlaugen-Massendurchflusses vorhanden ist.

5. Verfahren gemäss einem der Ansprüche 1 bis 4, **dadurch gekennzeichnet, dass** die Mutterlauge bei einer Temperatur von 20 bis 100°C zugeführt wird.

6. Verfahren gemäss einem der Ansprüche 1 bis 5, **dadurch gekennzeichnet, dass** das 2-Alkyl-1,3-propandiol, 2,2-Dialkyl-1,3-propandiol, 2-Alkyl-2-hydroxyalkyl-1,3-propandiol oder 2,2-Dihydroxyalkyl-1,3-propandiol 2-Methyl-1,3-propandiol, Neopentylglykol, 2-Butyl-2-ethyl-1,3-propandiol, Trimethylolethan, Trimethylolpropan, Trimethylolbutan oder Pentaerythritol ist.

7. Verfahren gemäss einem der Ansprüche 1 bis 6, **dadurch gekennzeichnet, dass** die organischen Nebenprodukte zumindest ein geradkettiges oder cyclisches Formal eines 2-Alkyl-1,3-propandiols, eines 2,2-Dialkyl-1,3-propandiols, eines 2-Alkyl-2-hydroxyalkyl-1,3-propandiols oder eines 2,2-Dihydroxyalkyl-1,3-propandiols einschliessen.

8. Verfahren gemäss einem der Ansprüche 1 bis 7, **dadurch gekennzeichnet, dass** die organischen Nebenprodukte organometallische Salze, wie Kalium-, Natrium- und/oder Calciumformiat, einschliessen.

9. Verfahren gemäss einem der Ansprüche 1 bis 8, **dadurch gekennzeichnet, dass** ein Waschabschnitt als Teil der ersten Extraktionseinheit oder als getrennte Einheit eingeschlossen ist.

## Revendications

1. Procédé d'extraction dans un processus de production de 2-alkyl-1,3-propanediol, de 2,2-dialkyl-1,3-propanediol, de 2-alkyl-2-hydroxyalkyl-1,3-propanediol ou de 2,2-dihydroxyalkyl-1,3-propanediol, **caractérisé en ce que** ledit procédé comprend les étapes consistant :
i) à fournir une solution mère, obtenue dans un processus de production de 2-alkyl-1,3-propanediol; de 2,2-dialkyl-1,3-propanediol, de 2-alkyl-2-hydroxyalkyl-1,3-propanediol et/ou de 2,2-dihydroxyalkyl-1,3-propanediol, à un débit massique contrôlé à une première unité d'extraction ;
ii) à extraire ladite solution mère dans ladite première unité d'extraction dans au moins un solvant à point d'ébullition élevé, où l'au moins un solvant à point d'ébullition élevé est l'éther de diisopropyle, le 2-éthylhexanol, le 2-propylheptanol, l'acétate d'éthyle, l'octanol, l'acétate de butyle, l'acétate d'isopropyle, la méthyléthylcétone et/ou la méthylisobutylcétone et est présent en une quantité supérieure à 0,2 fois le débit massique de ladite solution mère ;
iii) à obtenir un raffinat et un extrait comprenant ledit solvant et des sous-produits organiques ;
iv) à recycler ledit raffinat dans ledit processus ;
v) à fournir, à un débit massique contrôlé l'extrait obtenu à une deuxième unité d'extraction ;
vi) à extraire ledit extrait dans l'eau étant présente en une quantité allant de 1/10 à 1 fois le débit massique dudit extrait ;
vii) à obtenir une phase aqueuse comprenant lesdits sous-produits organiques et une phase de solvant ; et
viii) à recycler ladite phase de solvant à l'étape (ii), et éventuellement
ix) à évaporer ladite eau afin de produire des sous-produits organiques secs.

2. Procédé selon la revendication 1, **caractérisé en ce que** ladite solution mère et/ou ledit extrait est fourni(e) par l'intermédiaire d'un réservoir tampon.

3. Procédé selon la revendication 1 ou 2, **caractérisé en ce que** ledit raffinat est recyclé dans ledit processus par l'intermédiaire d'un réservoir tampon.

4. Procédé selon l'une des revendications 1 à 3 **caractérisé en ce que** ledit solvant est présent en une quantité allant de 1 à 4 fois le débit massique de ladite solution mère.

5. Procédé selon l'une des revendications 1 à 4 **caractérisé en ce que** ladite solution mère est fournie à une température comprise entre 20 et 100°C.

6. Procédé selon l'une des revendications 1 à 5, **caractérisé en ce que** ledit 2-alkyl-1,3-propanediol, 2,2-dialkyl-1,3-propanediol, 2-alkyl-2-hydroxyalkyl-1,3-propanediol ou 2,2-dihydroxyalkyl-1,3-propanediol est le 2-méthyl-1,3-propanediol, le néopentylglycol, le 2-butyl-2-éthyl-1,3-propanediol, le triméthyloléthane, le triméthylolpropane, le triméthylolbutane ou le pentaérythritol.

7. Procédé selon l'une des revendications 1 à 6 **caractérisé en ce que** lesdits sous-produits organiques comportent au moins un formal linéaire ou cyclique de 2-alkyl-1,3-propanediol, de 2,2-dialkyl-1,3-propanediol, de 2-alkyl-2-hydroxyalkyl-1,3-propanediol ou de 2,2-dihydroxyalkyl-1,3-propanediol.

8. Procédé selon l'une des revendications 1 à 7 **caractérisé en ce que** lesdits sous-produits organiques comportent des sels organométalliques, tels que le potassium, le sodium et/ou le formiate de calcium.

9. Procédé selon l'une des revendications 1 à 8 **caractérisé en ce qu'**une section de lavage est incluse en tant que partie de la première unité d'extraction ou en tant qu'unité séparée.
